# EUROPEAN PATENT SPECIFICATION

(11) **EP 4 376 935 B1**
(45) Date of publication and mention of the grant of the patent: **01.04.2026**
(21) Application number: 22751767.9
(22) Date of filing: 29.07.2022
(51) Int. Cl.: A61N 1/04, A61F 5/40

(54) **FASTENING TAPE TO AID PATIENT RECOVERY**
BEFESTIGUNGSBAND ZUR UNTERSTÜTZUNG DER PATIENTENERHOLUNG
BANDE DE FIXATION POUR AIDER À LA GUÉRISON D'UN PATIENT

(30) Priority: 30.07.2021 US 202117390507
(43) Date of publication of application: 05.06.2024
(73) Proprietor: 6D Tape Inc., 04130 Sipoo (FI)
(72) Inventor: TASKINEN, Tapani, 02230 Espoo (FI)
(74) Representative: Boco IP Oy Ab
(86) International application number: PCT/FI2022/050508
(87) International publication number: WO 2023/007059

(56) References cited:
- WO-A1-2015/183690
- WO-A1-2019/072531
- WO-A1-2021/080867
- US-A1- 2018 104 087

## Description

### BACKGROUND

In general, therapeutic treatment to aid patients' recovery from bodily injuries (e.g., strained, damaged, or weakened muscles, torn and/or strained ligaments, bruising, and/or the like) and/or medical procedures (e.g., surgical procedures, such as joint replacement), and/or to minimize patient discomfort from disabilities and/or other conditions (e.g., Fibromyalgia, Multiple Sclerosis (MS), and/or the like) involves professional-guided treatment sessions (e.g., under the guidance of a physician, physical therapist, and/or the like) and/or patient-guided treatment sessions (e.g., self-guided exercises) that may be prescribed by the overseeing professional. While the professional-guided treatment sessions may involve an exercise portion in which the patient performs exercises similar to those included in the patient-guided treatment sessions, professional-guided treatment sessions may include additional therapeutic massage and/or manipulation sessions in which the professional manipulates the patient's body at or near the injury site in order to stimulate blood flow, minimize scar tissue formation, encourage muscle growth, and/or the like.

Historically, patients have been unable to recreate the therapeutic manipulation treatment offered by professionals between and/or after professional treatment sessions. Accordingly, a need exists for devices enabling patients to self-perform therapeutic manipulation treatment to further aid in recovery from bodily injury. US 2018/104087 Al discloses a fastening tape.

### BRIEF SUMMARY

Various embodiments are directed to a fastening tape (such as a therapy e.g., a medical therapy tape, physiotherapy tape, physical therapy tape, chiropractic therapy tape, naprapathic therapy tape, massage therapy tape, lymphatic therapy tape, sports therapy tape, and/or the like) having one or more fastening points ( which can be e.g. handles) and/or one or more stimulators configured to enable a patient to continue manipulation and/or massage based treatment without the supervision of a medical professional. For example, the patient may be able to lift and/or move portions of the patient's skin to encourage rehabilitation and/or healing of portions of the patient's body onto which the tape has been secured. For example, a therapy tape may be configured to aid in treatment of strained, damaged, and/or weakened muscles and/or ligaments, hematoma, bruising, cording, spinal injuries, numbness, tissue and/or muscle tension and/or stiffness (e.g., from spasticity) . The therapy tape may additionally be configured to aid in treatment of various bodily conditions, such as epicondylitis, plantar fasciitis, MS, fibromyalgia, swelling lymphedema, lipedema, and/or the like. In various embodiments, the therapy tape may be configured to aid in recovery from medical procedures (e.g., surgical procedures), such as dental procedures, plastic surgery, scar treatment, liposuction, and/or the like. The therapy tape may additionally be configured to provide treatment to prevent injury, to aid in comfort of the patient, and/or the like. For example, the therapy tape may be configured to trigger acupuncture points, pressure points, and/or the like. The therapy tape may additionally be configured to lift, stretch, and/or move tissue and/or anchor filaments connected with the tissue. The therapy tape may additionally be configured to provide support to various bodily portions (e.g., a limb), and/or the like. The therapy tape may be usable with adults, children, elderly patients, and/or the like. Moreover, in certain embodiments, the therapy tape may be usable with animals (e.g., horses, dogs, cats, and/or the like).

Various embodiments are directed to a fastening tape system comprising: one or more fastening tapes, each of the one or more fastening tapes comprising: a backing layer configured to conform to a portion of a patient's body, wherein the flexible backing layer defines a top side and a bottom side opposite the top side; and an adhesive material secured relative to the bottom side of the flexible backing layer, wherein the adhesive material is configured to adhere the backing layer against the patient's skin; wherein the adhesive material is configured to maintain adherence with the backing layer and the patient's skin; at least one stimulator configured to apply a stimulating signal to the patient's skin, wherein the stimulating signal is configured to cause a mechanical movement of the patient's skin in at least one dimension; and one or more fastening means at least partially secured relative to the one or more fastening tapes.

In various embodiments, the one or more fastening means may comprise one or more handles secured relative to the top side of the flexible backing layer, wherein the one or more handles are secured to the flexible backing layer via one or more fasteners. In various embodiments, the mechanical movement may comprise a back-and-forth movement in one of a horizontal direction and vertical direction along an axis in the one dimension. In various embodiments, the mechanical movement may be defined in two dimensions. In certain embodiments, the mechanical movement may comprise a mechanical rotational movement. Further, in certain embodiments, the stimulating signal may be configured to cause a vibration of the patient's skin. In certain embodiments, the at least one stimulator may comprise an electrical stimulator comprising a Transcutaneous Electrical Neuro Stimulator (TENS).

In various embodiments, the at least one stimulator may comprise a pressure applicator configured to cause a vertical movement in a vertical direction of an axis in one dimension or to cause a continuous pressure downwards. Further, in various embodiments, the one or more stimulator(s) may be fastened to the fastening tape at one or more fastening points. In certain embodiments, the at least one stimulator may be fastened to the one or more fastening tapes at one or more fastening points. In certain embodiments, one or both of the fastening tape and the at least one stimulator may have a curved profile corresponding at least in part to a profile of at least a portion of a patient's body.

In various embodiments, the one or more fastening means may be secured to the one or more fastening tapes by a fastener comprising adhesive. In various embodiments, the backing layer may be inelastic or elastic. In certain embodiments, the one or more fastening means may be configured for securing one or more of the at least one stimulators to the one or more fastening tapes. In certain embodiments, the adhesive material may be heat activated by the patient's body heat. In various embodiments, the one or more fasteners may comprise a second adhesive material different from said adhesive material, wherein the second adhesive material is configured to permanently secure the one or more handles relative to the top side of the flexible backing layer. In various embodiments, the one or more fasteners may comprise thread sewn through at least a portion of each of the one or more handles and the backing layer. In various embodiments, at least one of the one or more handles may comprise a single-ply flexible sheet secured relative to the backing layer.

Further, in certain embodiments, the one or more handles may comprise a base portion configured to be secured onto the top side of the backing layer and a grip portion extending away from the base portion, and wherein the base portion of the one or more handles are secured relative to the top side of the backing layer via the one or more fasteners. In certain embodiments, the backing layer may define a length and a width measured perpendicular to the length, wherein the length is substantially longer than the width, and wherein: at least one of the one or more handles extends across the backing layer in a direction parallel with the width of the backing layer. In various embodiments, the backing layer may define a length and a width measured perpendicular to the length, wherein the length is substantially longer than the width, and wherein: at least one of the one or more handles extends across the backing layer in a direction parallel with the length of the backing layer. In certain embodiments, the one or more handles may be detachably secured relative to the backing layer, and wherein the one or more fasteners are selected from: magnets, hook-and-loop fasteners, or snap-fasteners.

In various embodiments, the fastening tape system may further comprise a controller configured to generate one or more stimulator signals to selectively activate the at least one stimulator; wherein the controller comprises at least one communication interface configured to receive data transmitted from at least one external computing entity. In various embodiments, the communication interface may be a wireless communication interface. In various embodiments, the fastening tape system may further comprise at least one sensor configured for measurement of at least one property of the skin, wherein at least one sensor is further configured to transmit measurements results to the controller, and wherein the controller is configured to adjust the one or more stimulator signals based at least in part on the measurements results. In certain embodiments, the fastening tape system may further comprise at least one sensor configured for measurement of at least one property of the skin, wherein at least one sensor is further configured to transmit the measurements results to a mobile device, and wherein the controller is configured to adjust the one or more stimulator signals based at least in part on the measurements results. In various embodiments, at least a portion of the at least one stimulating signal may be programmed to start and stop automatically in accordance with a measurement signal of one or more of an oedema sensor and a strain gauge.

In certain embodiments, at least a portion of the at least one stimulating signal may be programmed either automatically or adjusted as a response to a sensor measurement for a certain duration, to be maintained for a certain duration at given intervals, and/or or for a number of durations. In various embodiments, the one or more sensors may include one or more of a strain gauge, an oedema sensor for measuring one or more of liquid content and fat content, a temperature sensor, an ultraviolet (UV) sensor, and infrared (IR) sensor, an audio sensor, a force sensor, a radio frequency (RF) sensor, an electrical sensor for measurement of one or more electrical properties, a mechanical sensor for measurement of one or more mechanical properties, a lymph flow sensor for measurement of lymph flow, a blood circulation sensor for measurement of blood circulation, an evaporation sensor for measurement of evaporation ability, a pH sensor, a position sensor for the rise of the skin, and an input sensor configured for registering an input signal given by the patient in order to increase or decrease the efficiency of a treatment for an adjustment of the treatment. Further, in various embodiments, the adhesive material may be secured to the backing layer such that the adhesive material and the backing layer do not substantially delaminate upon removal from the patient's skin.

Various embodiments are directed to a method for manipulating a flexible material, the method comprising: securing a fastening tape system against a surface of the flexible material, wherein the fastening tape system comprises: one or more fastening tapes, each of the one or more fastening tapes comprising: a backing layer configured to conform to a portion of the surface of the flexible material, wherein the flexible backing layer defines a top side and a bottom side opposite the top side; an adhesive material secured relative to the bottom side of the flexible backing layer, wherein the adhesive material is configured to adhere the backing layer against the surface of the flexible material; at least one stimulator configured to apply a stimulating signal to a flexible material, wherein the stimulating signal is configured to cause a mechanical movement of the flexible material in at least one dimension; and one or more fastening means for securing the at least one stimulator to the one or more fastening tapes; and applying a tensile force to the at least one of the fastening means to lift a portion of the backing layer and a secured portion of the surface of the flexible material.

In various embodiments, the flexible material may be a patient's skin. In various embodiments, the fastening means may comprise one or more handles being detachably secured relative to the top side of the flexible backing layer, and wherein the method further comprises steps for: securing one or more handles relative to the top side of the flexible backing layer via one or more detachable fasteners. In various embodiments, the adhesive material may be heat activated, and wherein securing the tape structure against a surface of the flexible material comprises: placing the adhesive material of the fastening tape system against the patient's skin; and raising the temperature of the adhesive material based on the patient's body heat to securely adhere the adhesive material to the patient's skin. In various embodiments, the backing material may be inelastic, such that applying the tensile force to at least one of the one or more handles causes at least a portion of the surface of the flexible material to displace in a direction of the tensile force by a distance at least substantially equal to a displacement of a handle.

In various embodiments, the method may further comprise: collecting data comprising measurement results relating to a manipulation; and analyzing the data by one or more of artificial intelligence and a statistical method in order to define parameters for individual treatments based at least in part on the measurement results.

In various embodiments, the adhesive material is secured to the backing layer such that the adhesive material and the backing layer do not substantially delaminate upon removal from the patient's skin. Thus, the inventive fastening tape of the invention, which can be a therapy tape, enables a tissue mobilization and an active therapeutic care in at least one dimension, and even up to six dimensions. The mechanical movement can e.g. take place only in one dimension back and forth along one axis, in two dimensions over a surface (the movement being describe by two axes), or in three dimensions (the movement being describe by three axes, such as x, y and z axes). When rotational movement is added around one, two or three axes a mobilization in more dimensions is achieved. The fastening tape system of the invention can be fastened to directly on the skin or it can be formed in the form of a bracelet, or it can be fastened to the clothes of a person or the like.

### BRIEF DESCRIPTION OF THE SEVERAL VIEWS OF THE DRAWINGS

Reference will now be made to the accompanying drawings, which are not necessarily drawn to scale, and wherein:
Figures 1A-1C show top and side views of various therapy tape embodiments;
Figures 2A-2D show top and side views of various therapy tape embodiments;
Figure 3 shows a top view of an example therapy tape embodiment;
Figures 4A-4D show top and side views of various therapy tape embodiments;
Figure 5 shows a top view of a therapy tape comprising various stimulators according to one embodiment;
Figures 6A-6B show top and side views of a therapy tape comprising stimulators according to one embodiment;
Figure 6C shows an exploded view of a stimulator and handle according to one embodiment;
Figure 7 shows a side view of an example therapy tape comprising stimulators secured against a patient's skin according to one embodiment;
Figure 8 shows a side view of an example therapy tape;
Figures 9a and 9b show a top view and a side view, respectively, of an example therapy tape comprising a mechanical stimulator according to a first embodiment;
Figures 10a and 10b show a top view and a side view, respectively, of an example therapy tape comprising a mechanical stimulator according to a second embodiment;
Figure 11 shows a top view and a side view of an example therapy tape comprising a mechanical stimulator according to a third embodiment;
Figure 12 shows a top view and a side view of an example therapy tape comprising a mechanical stimulator according to a fourth embodiment;
Figure 13 shows a top view and a side view of an example therapy tape comprising a mechanical stimulator according to a fifth embodiment; and
Figure 14 shows a top view of an example therapy tape comprising a mechanical stimulator according to a sixth embodiment.

### DETAILED DESCRIPTION

The present invention will now be described more fully hereinafter with reference to the accompanying drawings, in which some, but not all embodiments of the invention are shown. Indeed, the invention may be embodied in many different forms and should not be construed as limited to the embodiments set forth herein. Rather, these embodiments are provided so that this disclosure will satisfy applicable legal requirements. Like numbers refer to like elements throughout. In the following, 1 inch = 2,54 cm.

In various embodiments, the fastening tapes of the invention comprise stimulators (e.g., vibrators, pressure applicators, transcutaneous electrical nerve stimulation devices (TENS devices), mechanical devices for movement and rotation, and/or the like and/or combinations of those) to facilitate therapeutic treatment of a patient injury, disability, medical treatment site, and/or the like; fastening points, to which the stimulator(s) is or are fastened. A fastening point can e.g. be a hook, a magnetic mechanism or one or more handles (e.g., detachable and/or secured relative to a backing layer), and/or sensors, (e.g. a strain gauge, an oedema sensor for measuring liquid content or fat content, a temperature sensor, an ultraviolet (UV) sensor, and infrared (IR) sensor, an audio sensor, a force sensor, a radio frequency (RF) sensor, a sensor for measurement of electrical properties, such as impedance, capacitance, resistance, reactance, or inductance, a sensor for measurement of mechanical properties, such as strength, elasticity, resilience, or flexibility, a sensor for measurement of lymph flow, a sensor for measurement of blood circulation, a sensor for measurement of evaporation ability, a pH sensor, a sensor for the rise of the skin, and/or a sensor with which it is possible to register a signal given by the patient in order to increase or decrease the efficiency of the treatment for an adjustment of the treatment.

In various embodiments, the method of the invention comprises steps for adjustment of a therapy on the basis of the measurement of the mechanic properties of the skin, wherein the adjustment is performed by a software program via a mobile device or a separate controlling unit on the basis of measurement results from the sensors. The connection can be wired or wireless, e.g. via a Bluetooth connection.

The therapy tape may comprise a backing material configured to support additional features of the tape, an adhesive layer configured to selectably secure the tape against a surface of a flexible material (e.g., a patient's skin or on clothes or as a band around the wrist), one or more fastening means, such as mechanical mechanisms, hooks, handles, and/or one or more stimulators. In various embodiments, handles may be integrated into the tape to enable a healthcare professional (e.g., physician, physical therapist, and/or the like), the patient, and/or another individual to manipulate the material (e.g., skin). For example, the handles may enable the healthcare professional and/or the patient to pull, stretch, twist, extend, elongate, and/or the like the underlying skin, tissue, fascia, and/or the like as a part of a medical treatment (e.g., a medical treatment including stretching, pulling, pushing, twisting, and/or the like the underlying skin, tissue and/or fascia of a patient) to encourage blood flow within and/or proximate the skin to which the tape is secured, to break-up and/or discourage scar tissue development, to encourage muscle development, to improve lymphatic flow and/or drainage, to increase fluid flow within a patient's body, to increase metabolic rate of a patient's body, and/or the like. Accordingly, the adhesive layer of the tape may be configured to be securely fastened against the patient's skin such that pulling and/or manipulating one or more handles of the tape does not cause the tape to detach from the patient's skin. Moreover, the therapy tape may be configured to vary in length and/or width, for example, as it is stretched, compressed, and/or the like.

Moreover, in various embodiments, the therapy tape may comprise one or more stimulators configured to stimulate the patient's skin while the tape is secured to the patient. In various embodiments, the therapy tape may comprise an integrated controller comprising a power supply and/or one or more control devices configured to selectably activate and/or deactivate the one or more stimulators. In various embodiments, the controller may be configured for wireless and/or wired connection with an external computing device (e.g., handheld computing device, a mobile device, desktop computing device, laptop computing device, control panel, controlling unit, and/or the like). The controller may be configured to transmit signals to the computing device indicative of current and/or historical status of the one or more stimulators, and/or may be configured to receive one or more control signals from the external computing entity configured to manipulate operation of the one or more stimulators.

In various embodiments, therapy tape may comprise a plurality of stimulators (e.g., a plurality of stimulators of the same type and/or a variety of types of stimulators) and/or one or more handles. Accordingly, the therapy tape may be configured to provide a variety of therapy types for the patient.

In various embodiments, the therapy tape may be configured to remain secured against a patient for an extended period of time (e.g., greater than one day). In such embodiments, the therapy tape may be configured to enable a patient to self-provide therapy when not in direct communication with a healthcare professional.

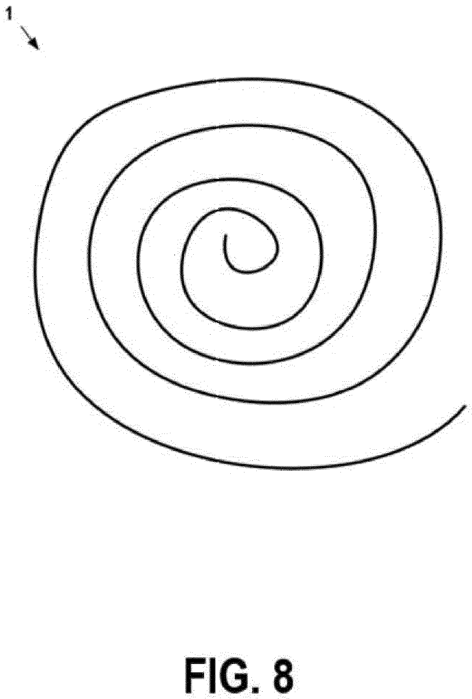

As discussed herein, the therapy tape may have a length and a width. In certain embodiments, the length of the therapy tape may be significantly longer than the width, such that the therapy tape may be rolled (e.g., onto a roll) for storage and/or distribution (shown in FIG. 8). As just one non-limiting example, the therapy tape may have a width of at least approximately 1-inch and a length of at least 6 inches (e.g., a length of 3-feet, 6-feet, 12-feet, 50-feet, 100-feet, and/or the like). As will be discussed in greater detail herein, the therapy tape may be configured to be stored on a roll, and a length of tape may be removed and detached (e.g., cut) from the roll for use. However, it should be understood that the therapy tape may have any proportion and/or relative dimensions. For example, in certain embodiments, the width may be larger than the length. Moreover, in certain embodiments, the therapy tape may vary in length (e.g., across the width of the tape) and/or width (e.g., across the length of the tape).

### Backing Layer

In various embodiments, a therapy tape may comprise a backing layer having an adhesive layer secured thereto. In certain embodiments, the backing layer may additionally have one or more handles and/or one or more stimulators secured thereto.

The backing layer may comprise a woven or nonwoven material, such as a woven fabric, a nonwoven film, a nonwoven fabric, and/or the like. In various embodiments, the backing layer may comprise a single layer, however in certain embodiments, the backing layer may comprise a plurality of layers (e.g., a woven layer and a non-woven layer). In certain embodiments, the backing layer may comprise a plurality of fibers (e.g., woven fibers, blown fibers, and/or the like). The plurality of fibers may comprise reinforcing fibers having a high tensile strength and configured to impede undesirable tearing and/or breaking of the tape. Moreover, in certain embodiments, the plurality of fibers may comprise elastic fibers configured to enable the backing layer to reversibly stretch in one or more directions.

In various embodiments, the backing layer may comprise one or more natural and/or synthetic materials. For example, the backing layer may comprise plant-based materials (e.g., cotton, wood fibers, bamboo fibers, cellulose fibers, natural rubber, and/or other biodegradable materials) synthetic materials, (e.g., polyester, synthetic rubber, polyvinyl chloride, and/or the like), and/or the like. In various embodiments, the backing layer may be hydrophobic, such that the backing layer may dry quickly when exposed to a moist environment (e.g., sweat, water, and/or the like). However, in certain embodiments, at least a portion of the backing material may be hydrophilic. As specific examples, the backing layer may comprise a Kinesiology tape backing layer, a medical support tape backing layer, an athletic tape backing layer, a dynamic tape backing layer, and/or the like. In various embodiments, the backing layer may comprise a plurality of materials. For example, a first portion of a backing layer may comprise a first material and a second portion of the backing layer may comprise a second material. As a specific example, a first portion of the backing layer may comprise a flexible, inelastic material and a second portion of the backing layer may comprise a flexible, elastic material.

In various embodiments, the backing layer may be a porous material, having a plurality of pores extending therethrough between a top surface and a bottom surface. For example, the pores may extend between fibers of a woven material, and/or through openings within a nonwoven material. Accordingly, the backing layer may be breathable, thereby allowing gases (e.g., air) to flow through the backing layer. Accordingly, the backing layer may enable air to flow to and/or away from a patient's skin located adjacent the therapy tape when secured thereto.

In various embodiments, the backing layer may be stretchable and/or elastic in one or more directions. For example, the backing layer may be stretchable and/or elastic in a direction parallel to the length of the tape and/or in a direction parallel to the width of the tape. Accordingly, in certain embodiments, the backing may be configured to provide a tensile force to a patient's skin when the therapy tape is secured thereto. For example, the elastic backing layer (and accordingly the remainder of the therapy tape) may be stretched during application to a patient and released once applied. Accordingly, the therapy tape may thus apply a tensile force as a result of the stretched elastic backing layer attempting to return to its original, unstretched form. However, it should be understood that in certain embodiments, the backing layer may be inelastic and may resist stretching (e.g., elongating). As will be discussed in greater detail herein, embodiments utilizing an inelastic backing layer may provide direct feedback to a patient's skin when manipulating the therapy tape (e.g., via handles). For example, because the backing layer does not stretch, displacing a portion of the therapy tape (e.g., via a handle) causes an at least substantially equal displacement of skin to which the therapy tape is secured.

### Adhesive Layer

As discussed herein, the therapy tape may comprise an adhesive layer secured to the backing layer. The adhesive layer may be configured to selectably secure the therapy tape against a patient's skin. Accordingly, the adhesive layer may be secured against the backing layer such that the adhesive layer and backing layer do not delaminate during application and/or removal from the patient.

In various embodiments, the adhesive layer may be secured against the backing layer via any of a variety of processes, as discussed in greater detail herein. For example, the adhesive layer may comprise an adhesive sheet laminated against the backing layer, an adhesive spray sprayed onto the backing layer, an adhesive liquid rolled onto the backing layer, dripped onto the backing layer, and/or the like.

In various embodiments, the adhesive layer may be continuous, such that at least substantially an entire surface of the backing layer is covered with the adhesive layer. However, in certain embodiments, the adhesive layer may be discontinuous, and may thus comprise a plurality of spaced adhesive portions secured to a surface of the backing layer. For example, the adhesive layer may comprise a plurality of spaced rectangular, circular, triangular, and/or the like adhesive portions therein. In various embodiments, the adhesive layer may be breathable (e.g., through adhesive portions and/or between adhesive portions) such that gases (e.g., air) may flow through the backing layer and the adhesive layer.

Like the backing layer, the adhesive layer may be elastic and/or stretchable, such that the elastic layer may move and/or stretch with the backing layer. Accordingly, the adhesive layer may be configured to conform with the movement of the backing layer such that the adhesive layer does not provide any force relative to the backing layer that may cause the adhesive layer to delaminate from the backing layer.

In various embodiments, the adhesive layer may be configured to detachably secure the therapy tape against a patient's skin. In various embodiments, the adhesive layer may provide sufficient tensile strength between the therapy tape and the patient's skin that manipulating the therapy tape (e.g., via handles) may cause relative manipulation of the patient's skin, and the therapy tape may remain secured to the patient's skin. In various embodiments, the adhesive layer may have a low shear strength to facilitate removal of the therapy tape from the patient, such as by stretching the therapy tape relative to the patient's skin. In various embodiments, the adhesive may comprise an acrylic polymer. As just one non-limiting example, the adhesive layer may comprise Stratagel^{®} adhesive material offered by Nitto Denko.

In certain embodiments, the adhesive layer may be heat-activated to enable the therapy tape to be secured to a patient's skin and/or to be removed from the patient's skin. Accordingly, the therapy tape may be secured to a patient's skin, and the patient's body heat may sufficiently heat the therapy tape to activate the adhesive layer such that the tape is secured relative to the patient's skin. Once activated and secured to the patient's skin, the therapy tape may be manipulated together with the patient's skin (e.g., via handles) without the therapy tape become dislodged from the patient's skin.

### Handle

With reference to the figures, the therapy tape may comprise one or more handles secured relative to the backing layer to enable lifting of the therapy tape and the underlying flexible material (e.g., skin) to which it is secured. The therapy tape may have a single handle and/or a plurality of handles having any of a variety of configurations. In various embodiments as discussed herein, the handles may be hook-shaped, circular, "U"-shaped, "D"-shaped, and/or the like. For example, in various embodiments handles may be O-rings, U-rings, J-rings, D-rings, and/or the like. Moreover, in various embodiments, the handles of the therapy tape may be secured relative to the backing layer such that the handle concentrates tensile forces applied to the handle along a center portion of the backing layer in order to impede peeling of the tape from the applied patient's skin.

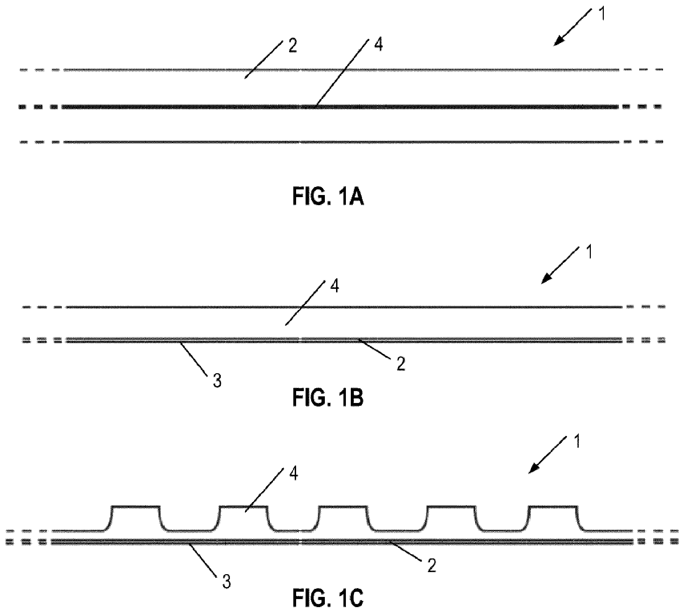

As shown in Figures 1A-1C, the therapy tape **1** comprises one or more handles **4** oriented at least substantially parallel with the length of the tape **1.** As shown in Figure 1A, which shows a top view of the tape **1,** the therapy tape **1** may comprise a backing layer **2** and a handle **4** positioned at least substantially centrally with the therapy tape **1.** As will be discussed herein, the handle **4** may be continuous with the backing layer **2,** and may be defined by a folded portion of the backing layer **2.** However, in various embodiments, the handle **4** may be a separate component from the backing layer **2** that is secured relative to the backing layer **2** with one or more fasteners (e.g., sewn threads, adhesive, knitting, magnets, zippers, ties, laces, rivets, and/or the like). In various embodiments, the handle **4** may comprise a flexible, inelastic, and/or elastic material (e.g., fabric, plastic, and/or the like) to enable the handle **4** to pivot and/or otherwise flex relative to the backing layer **2.** In certain embodiments, the handle **4** may comprise the same material as the backing layer **2,** although in certain embodiments, the handle **4** may comprise a material different from the backing layer **2.** However, it should be understood that in certain embodiments, the handle may comprise a rigid material (e.g., wood, metal, plastic, and/or the like). For example, the handle **4** may comprise a Kinesiology tape material, a medical support tape material, an athletic tape material, a dynamic tape material, and/or the like. As yet other examples, the handle **4** may comprise any of the materials provided as example backing layer materials discussed herein. Moreover, in certain embodiments, the handle may comprise multiple materials. For example, a first portion of a handle may comprise a first material and a second portion of a material may comprise a second material. For example, a first portion of a handle may comprise a flexible, inelastic material, and a second portion of the handle may comprise a flexible, elastic material.

As shown in Figure 1B, which illustrates a side view of the therapy tape **1,** the handle **4** may extend away from the backing layer **2** (e.g., at least substantially perpendicular from the backing layer **2).** The handle **4** may be secured to a top side of the backing layer **2,** opposite a bottom side of the backing layer **2** to which the adhesive layer **3** is secured. As discussed herein, the adhesive layer may be configured to detachably secure the therapy tape **1** relative to a patient's skin.

As shown in Figures 1B and 1C, the handle **4** may be continuous along the length of the tape **1** and/or discontinuous along the length of the tape **1.** As discussed herein, the handle **4** may be secured relative to the backing layer **2** such that tensile forces applied to the handle are concentrated along a central portion to impede delamination of the therapy tape **1** from the patient's skin while a tensile force is applied to the patient's skin. For example, then handles **4** may comprise one or more materials configured to concentrate a tensile force along a central portion of the therapy tape **1.** In various embodiments, the handle **4** may be configured to distribute a tensile force along at least a portion of the tape in a linear distribution, a parabolic distribution, and/or the like. For example, the tensile force may be concentrated in a central portion of the tape and the amount of tensile force applied to the tape may decrease (e.g., linearly, exponentially, hyperbolically, and/or the like) approaching the edge of the tape. The handle **4** may comprise one or more elongated portions extending away from the backing layer **2** and/or one or more short portions extending away from the backing layer **2.** The elongated portions and/or the short portions may have any of a variety of shapes, such as rectangular, elliptical, half-circular, triangular, and/or the like. In various embodiments, the relative shape and/or positioning of the handle portions may be determined during and/or after application to a patient's skin. For example, a handle elongated portion may be defined (e.g., via cutting and/or securing to the backing layer **2)** proximate a point of interest in the patient's skin (e.g., a proximate point of injury).

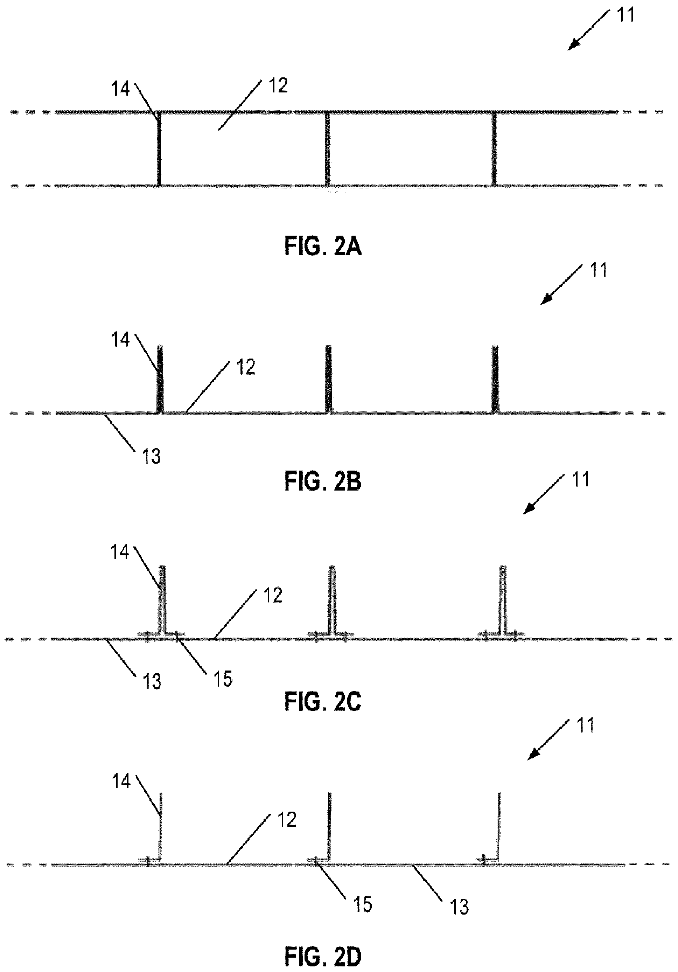

With reference now to Figures 2A-2D, a therapy tape **11** may comprise a plurality of handles **14** oriented at least substantially perpendicular to the length of the therapy tape **11.** In various embodiments, the handles **14** may extend between at least approximately 0.001 inches-48 inches away from the backing layer. As just one example, the handles may extend between about 0.5 inches-6 inches away from the backing layer. As a specific example, the handles may extend between about 1 inch-2 inches away from the backing layer. Therapy tape **11** may have a similar configuration to therapy tape **1** (e.g., materials, handle securing fasteners, and/or the like), however therapy tape **11** may have a different handle orientation relative to the length of the therapy tape **11.** As shown in Figures 2A-2D, therapy tape **11** may comprise handles **14** extending away from a top surface of backing layer **12** (opposite adhesive layer **13),** and at least substantially perpendicular to the length of therapy tape **11.** As shown in Figure 2B, the handles **14** may be formed from looped portions of the backing layer **12** extending upwardly away from a primary plane of the backing layer **12** (e.g., plane against which the patient's skin is positioned). The looped portion of the backing layer **12** may be fastened together (e.g., by sewn thread, by adhesive, and/or the like) to form handles **14.** In embodiments in which adhesive is utilized to secure form the handles **14** utilizing lengths of looped backing layer **12,** the adhesive utilized to securely form the handles **14** may be different from the adhesive utilized to secure the therapy tape against a patient's skin.

Figures 2C-2D illustrate alternative embodiments for securing handles **14** relative to backing layer **12.** As shown in Figure 2C, the handle **14** may comprise a base portion configured to be secured adjacent and/or parallel with a backing layer, and a grip portion extending away from the base. The base portion may comprise one or more material layers (e.g., a single layer) and the grip portion may comprise at least the same number of material layers as the base portion (e.g., a single layer or two layers). In various embodiments, the handles **14** may comprise a folded material secured relative to the backing layer **12.** In such embodiments, the folded layers of the handle **14** may form the base portion and the grip portion such that the base portion may be secured relative to the backing layer, and the folded portion forming the grip portion may be secured together. For example, one or more folded layers forming the grip portion may be secured relative to one another using a fastener as discussed herein (e.g., sewn thread, adhesive, and/or the like). As shown in Figure 2C, the base portion of the folded layers may be secured relative to the backing layer **12** using one or more fasteners (e.g., sewn thread, adhesive, and/or the like). In various embodiments, the base portion may define an enlarged area to be secured against the backing layer. Accordingly, when a tensile force is applied to the handle (e.g., grip portion) as discussed herein, the enlarged base portion may serve to distribute the effect of the tensile force across an area of the backing layer, thereby distributing the tensile force across a larger area of the patient's skin to discourage the backing layer from becoming dislodged from the patient's skin during treatment. For example, in embodiments in which the handles **14** are at least substantially aligned perpendicular to the length of the backing layer **12,** the base portion may have a width at least substantially equal to the backing layer **12,** and a length of at least 1 inch. In various embodiments, the base portion may have a length between 1 inch and 12 inches.

In embodiments in which the handles **14** are oriented at different angles relative to the backing layer **12,** the base portion of the handles may be positioned entirely adjacent the backing layer **12,** such that no portion of the base portion extends beyond the edges of the backing layer **12.** In certain embodiments, the base layer may have a substantially equal size and shape as the backing layer **12.**

Figure 2D shows yet another example embodiment for securing handles **14** relative to backing layer **12.** As shown in Figure 2D, each handle may comprise a single layer of material (e.g., fabric, plastic, and/or the like) secured relative to the backing layer **14** with one or more fasteners (e.g., sewn thread, adhesive, and/or the like).

In various embodiments, handles **14** may be formed and/or secured relative to backing layer **12** during and/or after securing the therapy tape **11** relative to a patient's skin. Accordingly, the handles **14** may be positioned by a user (e.g., a healthcare professional) based on therapeutic needs of the patient. For example, the handles **14** may be secured relative to the therapy tape **11,** and therefore relative to the patient, based on the location of an injury, and/or the like.

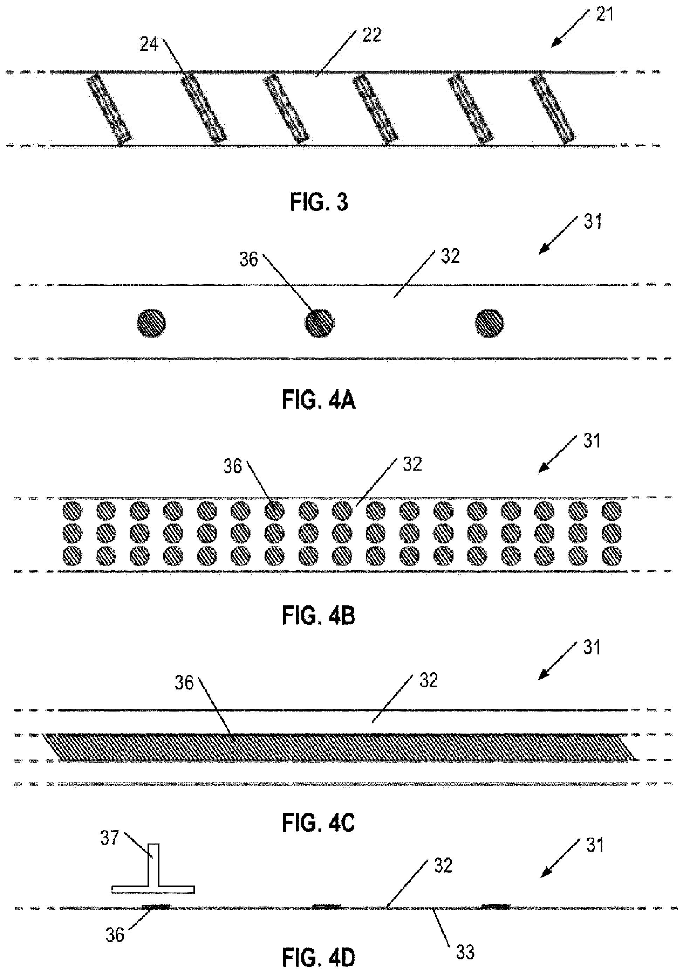

As shown in Figure 3, which shows a therapy tape **21** having a configuration similar to therapy tapes **1** and **11** discussed here. Therapy tape **21** may comprise a backing layer **22** to which one or more handles **24** are secured. As shown in Figure 3, handles **24** may be secured at any orientation relative to the backing layer **22.** The handles **24** may be secured using any of a variety of configurations as discussed herein (e.g., formed as folded portions of backing layer **22,** formed as folded portions of material secured relative to backing layer **22,** formed as single layers of material secured relative to backing layer **22,** and/or the like).

With reference to Figures 4A-4D, therapy tape **31** may be configured such that handles may be detachably secured relative to backing layer **32.** In the illustrated embodiments of Figures 4A-4D, backing layer **32** and adhesive layer **33** may have a configuration similar to those described in reference to therapy tapes **1, 11,** and **21** above. However, as shown in Figures 4A-4D, therapy tape **31** may additionally comprise one or more connecting portions **36** secured relative to backing layer **32** that is configured to enable one or more handles **37** to be detachably secured relative to the therapy tape **31.** In various embodiments, connecting portions **36** may comprise any of a variety of detachable fastener materials, such as hook-and-loop material (e.g., Velcro^{®}), magnetic material, detachable and/or permanent adhesive, snaps, and/or the like. As just one example, the adhesive material of the adhesive layer **33** may be continued onto the backing layer **32,** such that handles **37** may be secured relative to the adhesive material on the backing layer.

As shown in Figures 4A-4D, the connecting portions **36** may comprise continuous connecting portions **36** (e.g., as shown in Figure 4C) and/or discontinuous connecting portions **36** (e.g., as shown in Figures 4A-4B). In various embodiments, each connecting portion **36** may be configured to be secured relative to a detachable handle **37.** For example, each connecting portion **36** may comprise a female snap connector configured to be secured relative to a male snap connector of a detachable handle **37.** In yet other embodiments, a plurality of connecting portions **36** may be configured to be secured relative to a single detachable handle **37.** For example, in the illustrated embodiment of Figure 4B, a plurality of connecting portions **36** may be configured to engage a detachable handle **37** simultaneously to secure the detachable handle **37** relative to the therapy tape **31.**

As shown in Figure 4D, the connecting portions **36** may have a low profile to avoid unintentionally snagging items placed adjacent the therapy tape **31** (e.g., clothes). Moreover, in various embodiments, the one or more connecting portions **36** may be configured to enable a detachable handle **37** to be secured relative to the therapy tape **31** through one or more additional layers (e.g., clothing layers). Thus, for example, the therapy tape **31** may be secured to a patient's skin under the patient's clothes, and a detachable handle **37** may be configured to engage to connecting portion **36** through the patient's clothes (e.g., via magnetic force) to enable the patient and/or healthcare professional to manipulate the therapy tape **31** and the secured patient's skin through the patient's clothes.

In various embodiments, connecting portions **36** may be secured relative to a top side of backing layer **32** (e.g., opposite adhesive layer **33).** In such embodiments, connecting portions **36** may be secured to the backing layer **31** via one or more fasteners (e.g., sewn thread, adhesive, rivet, and/or the like). In certain embodiments, connecting portions **36** may be secured adjacent bottom side of the backing layer **32** (e.g., between backing layer **32** and adhesive layer **33** or on an opposite side of the adhesive layer **33** relative to backing layer **32).** In such embodiments, the connecting portions **36** may be secured relative to the backing layer **32** via the adhesive properties of adhesive layer **33,** and/or via an additional fastener (e.g., an additional adhesive, sewn thread, and/or the like).

Like handles **4** and **14,** connecting portions **36** may be secured relative to the backing layer **32** during and/or after securing the therapy tape **31** relative to the patient. For example, the connecting portions **36** (e.g., magnets) may be placed between the adhesive layer **33** and the patient's skin, such that the connecting portions **36** are pinned between the therapy tape **31** and the patient's skin. As yet another embodiment, the connecting portions **36** may be secured relative to a top side of the backing layer **32** during and/or after securing the therapy tape **31** relative to the patient's skin.

In various embodiments, the detachable handle **37** may comprise a rigid component and/or a flexible component. For example, the detachable handle **37** may comprise a magnetic material configured to be magnetically secured relative to a magnetic connecting portion **36.** As yet another example, the detachable handle **37** may comprise a grip portion (e.g., a flexible grip portion and/or a rigid grip portion) configured to enable a user (e.g., patient and/or healthcare professional) to manipulate the detachable handle **37** and a connector configured to engage the connecting portion **36** of the therapy tape **31.** For example, the connector may comprise a mating hook-and-loop material configured to be secured relative to a corresponding hook-and-loop connecting portion **36.** As yet another example, the connector of the detachable handle **37** may comprise a snap-type connector (e.g., a male snap-type connector) configured to engage a corresponding snap-type connector (e.g., a female snap-type connector) of a connecting portion **36.**

In various embodiments, the therapy tape may comprise one or more handles (e.g., handles **4, 14, 24)** and one or more connecting portions **36** configured to detachably secure one or more detachable handles **37** relative to the therapy tape.

### Stimulators and Controller

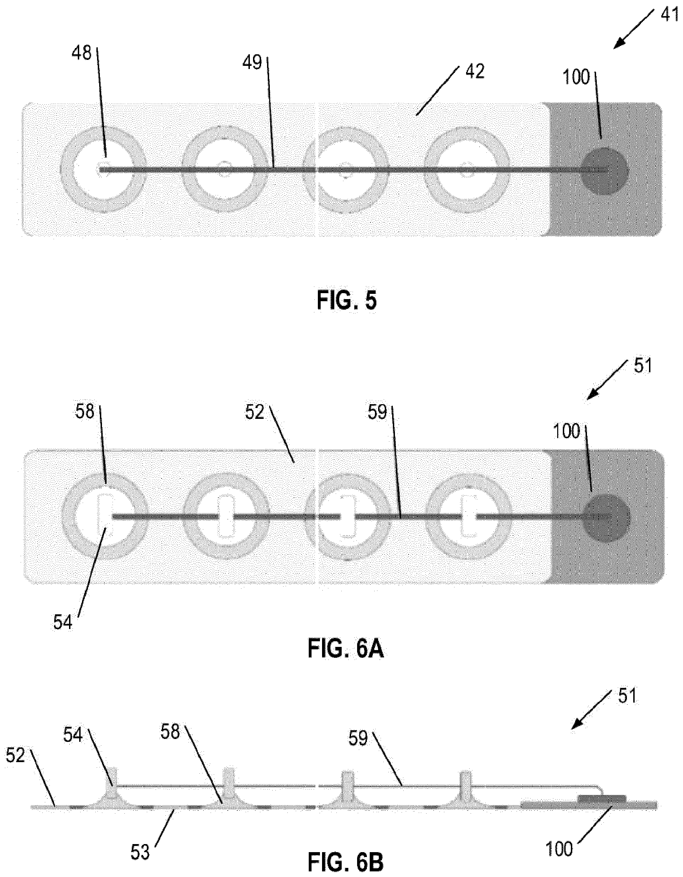

With reference to Figures 5-7, the therapy tape may comprise one or more stimulators (e.g., vibration element, pressure applicators, TENS devices, biofeedback devices, bioimpedance analysis devices, thermometers, pulse measurement devices, and/or the like) configured to provide stimulation to the skin to which the therapy tape is secured. As discussed herein, various embodiments of therapy tape may comprise a single vibration element, a plurality of vibration elements, a single pressure applicator, a plurality of pressure applicators, a single TENS electrode, a plurality of TENS electrodes, and/or the like. In various embodiments, a therapy tape may comprise one or more vibration elements and one or more pressure applicators. In yet other embodiments, a therapy tape may comprise one or more vibration elements and one or more TENS electrodes. In yet other embodiments, a therapy tape may comprise one or more pressure applicators and one or more TENS electrodes. In yet other embodiments, a therapy tape may comprise one or more vibration elements, one or more pressure applicators, and one or more TENS electrodes.

As shown in Figure 5, the therapy tape **41** may comprise a backing layer similar to that discussed herein, with one or more vibration elements **48** secured thereto. In various embodiments, the one or more vibration elements **48** may be secured relative to a top side of the backing layer **42,** a bottom side of a backing layer **42** (e.g., between backing layer **42** and adhesive layer (not shown) or opposite adhesive layer relative to the backing layer **42).** As yet another embodiment, the therapy tape **41** may define one or more apertures extending therethrough (e.g., through backing layer **42** and/or adhesive layer) in which the vibration elements **48** are secured.

The one or more vibration elements **48** may comprise one or more vibration actuators configured to emit vibration pulses to a patient's skin to provide therapeutic sensations for the patient (e.g., pain relief, numbing, increased metabolic rate, and/or the like). In various embodiments, the one or more vibration elements **48** may be in electrical communication with a controller **100** (e.g., via electrical conduit **49)** configured to emit power signals to each of the one or more vibration elements **48.** In various embodiments, electrical conduit **49** may comprise one or more wires (e.g., solid and/or stranded), one or more printed electrical connectors (e.g., printed onto backing layer **42),** and/or the like.

In various embodiments, the controller **100** may comprise a power supply (e.g., a battery) and a control circuit configured to emit signals to the one or more vibration elements **48.** In various embodiments, the controller 100 may additionally comprise a communication interface configured to communicate with one or more external computing entities (e.g., a handheld computing device, such as a Personal Digital Assistant, a smartphone, a tablet, a smartwatch, and/or the like; a personal computing entity, such as a laptop computing device, a desktop computing device, and/or the like; and/or a central computing device, such as a server, a web interface, and/or the like). In various embodiments, the communication interface may be configured to communicate with one or more external communication interfaces via a wired interface and/or a wireless interface (e.g., Bluetooth^{®}, Wi-Fi, Near Field Communication, LTE, 3G, and/or the like). In various embodiments, the communication interface may be configured to communicate via one or more networks, such as the Internet, an Intranet, and/or the like.

In various embodiments, the controller 100 may be configured to receive control signals from the external computing entity to control the one or more vibration elements 48. For example, the external computing entity may be configured to transmit signals indicative of a desired active/inactive state for the one or more vibration elements 48, a desired vibration frequency for the one or more vibration elements 48, and/or the like. For example, the external computing entity may be configured to generate and transmit the one or more control signals in response to receipt of user input indicative of desired operating characteristics of the vibration elements 48.

Although not shown, therapy tape **41** may additionally comprise one or more handles (e.g., similar to handles **4, 14, 24)** and/or connecting portions **36** configured to be detachably secured to one or more detachable handles **37.**

With reference now to Figure 6A-6C, a therapy tape **51** may comprise one or more pressure applicators **58** (e.g., powered suction cups, compression sleeves, and/or the like) configured to provide pressure (e.g., negative pressure and/or positive pressure) to skin to which the therapy tape is secured **51.** Each of the pressure applicators **58** may be in electrical and/or pneumatic communication with a controller **100** which may comprise a pump, an electrical actuator, and/or the like configured to provide pressure to each of the pressure applicators **58.** For example, a conduit **59** extending from controller **100** to one or more of the pressure actuators **58** may comprise an electrical conduit (e.g., a wire) configured to transmit signals to each of the one or more pressure applicators **58** to cause each of the pressure applicators **58** to activate and provide pressure to the patient's skin. For example, each of the pressure applicators **58** may comprise actuators configured to apply a pressure to the patient's skin. In certain embodiments, the conduit **59** may comprise a pneumatic conduit (e.g., a tube) connecting a pump located at the controller **100** to each of the one or more pressure applicators **58.** In such embodiments, the pump may be configured to pump air from the pressure applicators **58,** thereby causing the pressure applicators to provide a pressure to the patient's skin.

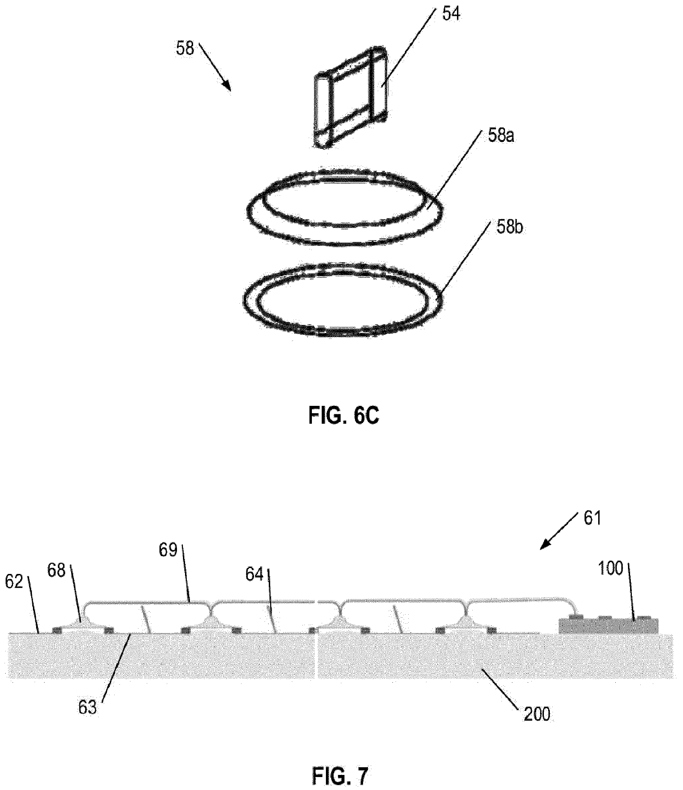

Figure 6B provides a side-view of a therapy tape **51** according to various embodiments. As shown in Figure 6B, the pressure applicators **58** may additionally comprise one or more handles **54** extending therefrom. Each of the one or more handles **54** may have a configuration similar to handles **4, 14,** and **24** as discussed herein. However, the handles **54** may be secured directly to the pressure applicators **58.** As shown in Figure 6C, handles **54** may extend from a top portion **58a** of the pressure applicators **58,** opposite a bottom portion **58b** configured to contact a patient's skin. Although not discussed in reference to the vibration applicators **48** discussed above, it should be understood that vibration applicators **48** may additionally comprise handles **54** as discussed herein.

With reference to Figure 7, various therapy tape **61** embodiments may comprise a plurality of stimulators **68.** As shown therein, therapy tape **61** may comprise a plurality of stimulators **68** comprising vibrating elements configured to apply vibration stimulation to the patient's skin, pressure applicators configured to apply pressure to the patient's skin, and/or transcutaneous electrical nerve stimulation devices (TENS devices) configured to apply electrical stimulation to a patient's skin. Each of the stimulators 68 may be in electrical and/or pneumatic communication with a controller **100** via a conduit **69.** As discussed herein, controller **100** may comprise one or more power sources, one or more electrical signal generators, and/or one or more pumps configured to actuate the one or more stimulators **68.** Moreover, as shown in Figure 7, the therapy tape **61** may comprise one or more handles **64** similar to handles **4, 14,** and **24.** Although not shown, therapy tape **61** may be configured to accept one or more detachable handles as discussed herein. In such embodiments, the therapy tape **61** may comprise one or more connecting portions **36** configured to engage one or more detachable handles **37.**

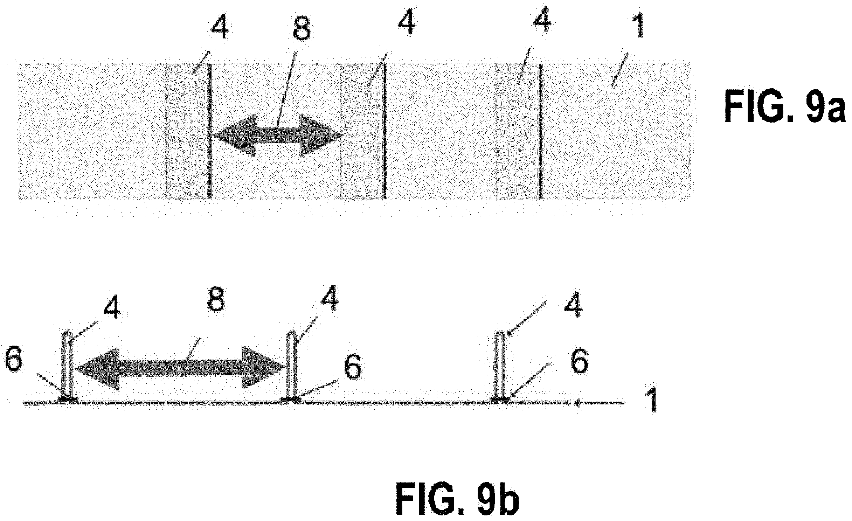

With reference to Figures 9a-14, an exemplary fastening tape system may comprise one or more fastening tapes 1, such as, for example, a therapy tape. Further, in various embodiments, a fastening tape system may comprise at least one stimulator fastened on one or more therapy tapes at one or more fastening points. For example, one or more of the at least one stimulator may comprise a mechanical stimulator. The stimulators do not need to have the form of these figures, but can have any form. In all these figures, reference number 8 represents the stimulator and reference number 1, the fastening tape, which can be a therapy tape. In various embodiments, one or more fastening tapes may define at least a portion of a fastening tape system. Further, in various embodiments, an exemplary fastening tape system may comprise at least one stimulator.

Figure 9a shows a top view and Figure 9b a side view of an example therapy tape 1 comprising a mechanical stimulator 8. As illustrated, a fastening tape such as therapy tape 1 may comprise one or more fastening means secured relative to the one or more fastening tapes. For example, in various embodiments, a fastening means may be configured for securing at least a portion of the at least one stimulator 8 to the one or more fastening tapes. As a non-limiting example, in various embodiments, a fastening means configured to secure a stimulator 8 to a fastening tape may comprise one or more fasteners.

In various embodiments, an example therapy tape 1 may comprise one or more handles 4 spaced along a length of the flexible backing layer of the therapy tape 1. As described herein, a handle may comprise an elongated material secured onto the top side of the flexible backing layer and extends across at least a portion of a width of the flexible backing layer perpendicular to the length of the therapy tape 1. In various embodiments, a fastening tape system may comprise a therapy tape 1 comprising a plurality of handles 4 defined at least in part by a first handle and a second handle, wherein a first end of the first handle is spaced a distance away from a second end of the second adjacent handle along the length of the flexible backing layer such that the first handle is discrete and separated from the second handle. As described herein, in various embodiments, one or more handles 4 may be secured to the flexible backing layer via one or more fasteners.

In various embodiments, the fastening of the stimulator 8 to the tape 1 is performed between two handles such that at least a portion of the at least one stimulator 8 is disposed along the length of the flexible backing layer within a gap between adjacent handles of the plurality of handles 4. As described herein, a stimulator may be configured to apply a stimulating signal to a flexible material, such as, for example, a patient's skin. For example, a fastening tape system may be configured such that a stimulating signal applied to a patient's skin by the at least one stimulator 8 may cause a mechanical movement of the patient's skin in at least one dimension. As described herein, a mechanical movement of a flexible material, such as, for example, a patient's skin, may comprise an at least temporary displacement of at least a portion of the flexible material defined by a translation, shift, rotation, and/or the like in one or more directions. In Figure 8a, the mechanical movement takes place in one direction back and forth in one dimension as indicated by the arrows.

In various embodiments, at least a portion of a handle 4 may be defined by a fastening means configured to secure one or more stimulators 8 relative to a fastening tape. As a non-limiting example provided for illustrative purposes, in various embodiments, a handle 4 secured to a fastening tape may include a fastening means embodied by a pocket that is configured to receive at least a portion of a stimulator 8 therein so as to secure the stimulators 8 relative to the fastening tape.

As a further, non-limiting example, in various embodiments, one or more fastening means configured for securing a stimulator 8 relative to one or more fastening tapes may be further configured for securing at least a portion of one or more handles to the one or more fastening tapes. For example, in such an exemplary circumstance, the one or more fastening means of an example therapy tape 1 may comprise an adhesive comprising a first adhesive portion configured to secure a stimulator 8 relative to one or more fastening tapes of therapy tape 1, and a second adhesive portion configured to secure a handle relative to the one or more fastening tapes.

The handles are in Figure 9a secured with seams 6 to the underlying therapy tape 1 as an example. A more usual alternative, however, is to use adhesive for the securing.

The mechanical stimulator 8 is in Figures 9a and 9b fastened at two handles 4 between them. In various embodiments, one or more of the at least one stimulators may comprise an electrical stimulator comprising a Transcutaneous Electrical Neuro Stimulator (TENS). In such an exemplary embodiment, the fastening tape system may be configured such that a TENS is disposed about the one or more fastening tape within a gap between adjacent handles of a plurality of handles secured relative to the fastening tape. Further, in various embodiments, the at least one stimulator 8 of a fastening tape system may comprise a plurality of stimulators. In various embodiments, the plurality of stimulators may be disposed about the one or more fastening tapes such that two or more stimulators of the plurality are positioned within a gap between adjacent handles of a plurality of handles. Alternatively, or additionally, in various embodiments, a plurality of stimulators may be disposed about the one or more fastening tapes such that a first stimulator of the plurality of stimulators is positioned within a first gap between a first pair of adjacent handles, and a second stimulator of the plurality of stimulators is positioned within a second gap between a second pair of adjacent handles.

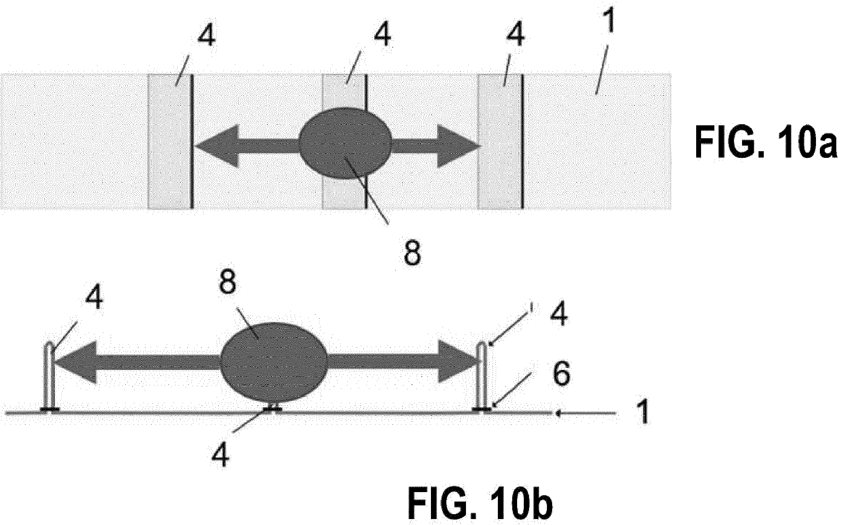

Figure 10a shows a top view and Figure 10b a side view of an example therapy tape 1 comprising a mechanical stimulator 8 according to a second embodiment, wherein the mechanical movement takes place in one direction back and forth in one dimension as indicated by the arrows.

The handles are in Figures 10a and 10b secured with seams 6 to the underlying therapy tape 1 as an example. In an alternative embodiment, however, a fastening tape system may comprise one or more fastening means, such as, for example, one or more handles may be configured such that an adhesive is used for securing the fastening means to a fastening tape. Further, as non-limiting examples provided for illustrative purposes, one or more stimulators of the fastening tape system 1 may be at least partially secured relative to one or more components of the fastening tape system 1, such as, for example, a fastening tape, a handle, and/or the like, using a clip, such as, for example, an alligator clip, a crocodile clip and/or any other suitable metal clip configured for permanent and/or temporary coupling to a portion (e.g., an end portion) of the stimulator; a Velcro^{®} material; a double-sided adhesive material; a single-sided adhesive material; an opening (e.g., defined by a sealing ring) configured to receive a hook element disposed about a portion (e.g., an end portion) of the stimulator, one or more snap elements, one or more magnetic elements (e.g., a magnetic material); one or more staples; one or more stich, seam, suture, and/or the like; one or more crimped connection elements; and/or the like.

The mechanical stimulator 8 is in Figures 10a and 10b fastened at three handles 4. In various embodiments, one or both of a fastening tape 1 and the at least one stimulator 8 may have an at least partially curved profile. The stimulator 8 in Figures 10a and 10b has an oval form with two arms, the fastening point at the middle handle 4 being at the oval portion of the stimulator 8. For example, in various embodiments, the curved profile of a fastening tape 1 and/or a stimulator 8 may correspond at least in part to a profile of at least a portion of a patient's body.

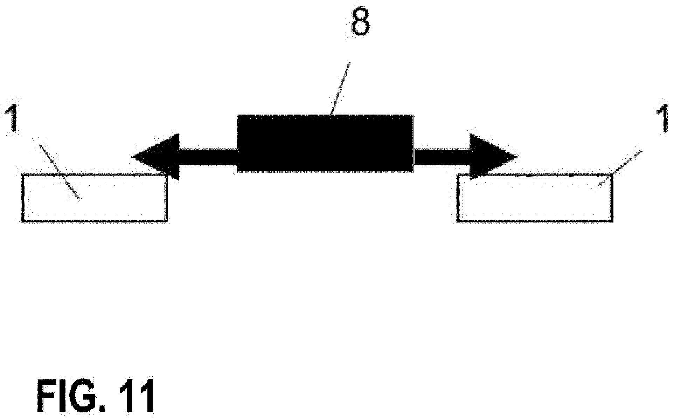

Figure 11 shows a side view of an example therapy tape system consisting of two therapy tapes 1 having a mechanical stimulator 8 thereon according to a third embodiment, wherein the mechanical movement takes place in one direction back and forth in one dimension as indicated by the arrows.

The mechanical stimulator 8 is in Figure 11 is fastened at two therapy tapes 1. The stimulator 8 in Figure 11 has a rectangular form with two arms, the fastening of which is at the two therapy tapes 1.

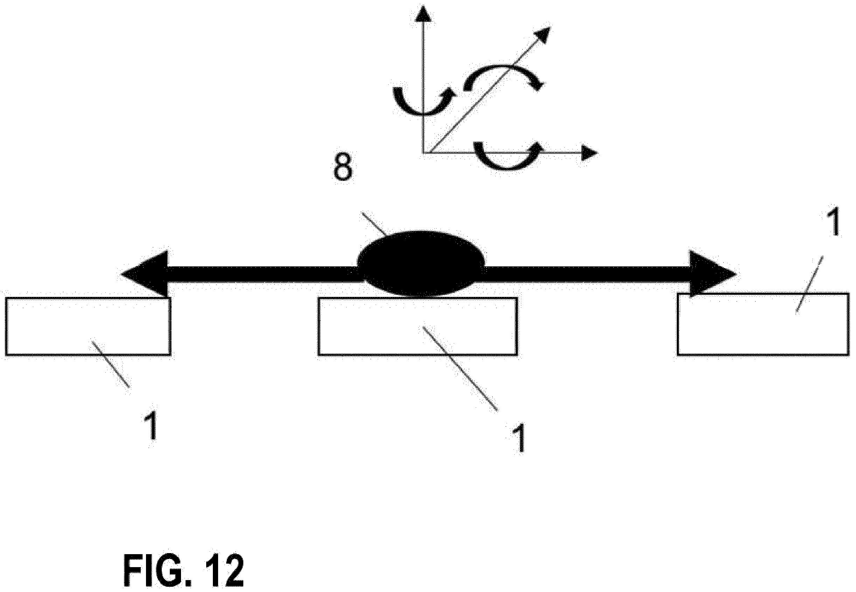

Figure 12 shows a side view of an example therapy tape system consisting of three therapy tapes 1 having a mechanical stimulator 8 thereon according to a fourth embodiment, wherein the mechanical movement takes place in one direction back and forth in one dimension as indicated by the arrows. In this embodiment the stimulator additionally causes a three-dimensional rotational movement, which is illustrated to take place around three axes, i.e. x-, y-, and z-axes, as indicated by the coordinate system in the figure and the rotation arrows.

The mechanical stimulator 8 is in Figure 12 is fastened at three therapy tapes 1. The stimulator 8 in Figure 12 has a rectangular form with two arms, the fastening of which is at the three therapy tapes 1 with the rectangular part at the middle therapy tape 1.

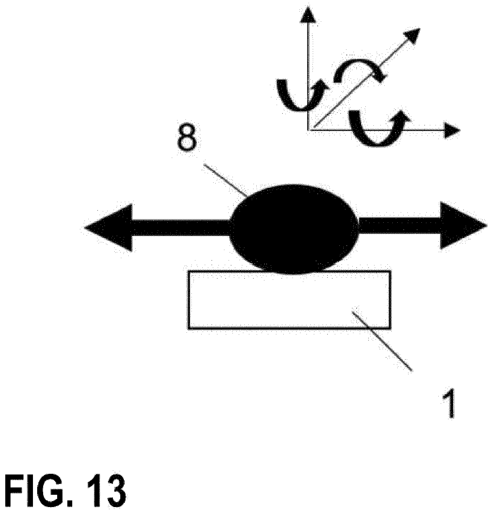

Figure 13 shows a side view of an example therapy tape 1 having a mechanical stimulator 8 thereon according to a fifth embodiment, wherein the mechanical movement takes place in one direction back and forth as indicated by the arrows. In this embodiment the stimulator additionally causes a three-dimensional rotational movement, which is illustrated to take place around three axes, i.e. x-, y-, and z-axes, as indicated by the coordinate system in the figure and the rotation arrows.

The mechanical stimulator 8 is in Figure 13 is fastened at two therapy tapes 1 at one fastening point. The stimulator 8 in Figure 13 has an oval form with two arms but can have any form as said above.

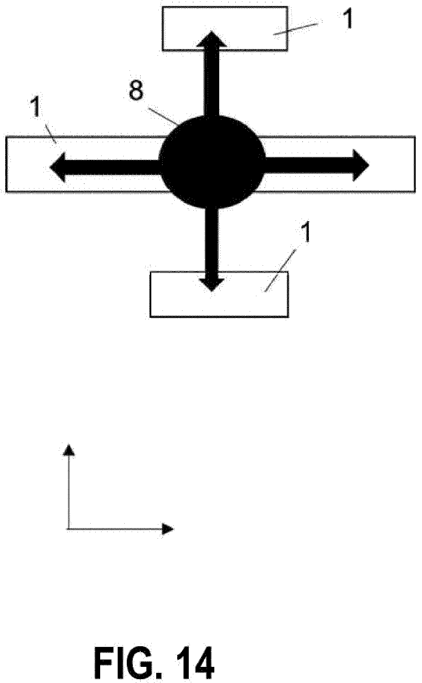

Figure 14 shows a top view of an example therapy tape comprising a mechanical stimulator according to a sixth embodiment, wherein the mechanical movement takes place two-dimensionally as indicated by the arrows, wherein there is a movement in the direction of the x-axis and a movement in the direction of the y-axis as indicated by the coordinate system in the figure and the arrows.

### Method of Manufacturing

In various embodiments, a therapy tape as discussed herein may be manufactured by forming and/or securing handles within and/or onto a backing material, and by applying an adhesive material to the backing material. Moreover, in various embodiments, a therapy tape may be formed as a portion of a large web (e.g., having a width equal to at least twice the width of the therapy tape) and the large web may be cut into individual therapy tape width portions. In certain embodiments, the therapy tape may be formed from a web of material at least substantially the same width as the therapy tape.

In various embodiments, the handles may be formed as a portion of the backing material by folding the backing material to form one or more loops therein. As discussed herein, the handles may align with the length of the therapy tape, and in such embodiments, the therapy tape may be contoured to form a loop extending above a top side of the backing material between lateral edges of the therapy tape. The looped portion of backing material may be fastened together (e.g., via an adhesive, sewn thread, rivets, and/or the like) to form one or more handles. In embodiments in which the handles extend laterally across the therapy tape between lateral edges of the therapy tape, a plurality of loops may be formed in the backing layer, for example, at various intervals (e.g., regular intervals and/or irregular intervals), and may be secured together with one or more fasteners.

A bottom side of the backing material, opposite the looped portion, may be coated with an adhesive material. In various embodiments, the adhesive material may be laminated onto the backing material, sprayed onto the backing material, screen printed onto the backing material, dripped onto the backing material, and/or the like. In certain embodiments, the adhesive material may be cured after being applied to the backing material, to form a strong mechanical bond between the backing layer and the adhesive material such that the adhesive material does not delaminate from the backing layer. After application of the adhesive material and formation of the handles, the therapy tape may be rolled onto a take-up roller for storage, shipping, and/or sale.

In various embodiments, the handles may be formed from a separate material and secured to the backing layer. For example, individual loops of material, strips of material, and/or the like may be secured to the backing layer via one or more fasteners (e.g., adhesive, sewn thread, rivets, and/or the like). For example, handles may be secured across the width of the therapy tape (e.g., extending between lateral edges). As yet another example, a handle may extend along the length of the therapy tape, and accordingly the handle may be secured along the length of the therapy tape.

In various embodiments, the adhesive material may be applied to the backing layer prior to forming the handles therein.

In embodiments comprising one or more connecting portions **36,** the connecting portions may be secured to the backing layer via one or more fasteners (e.g., glue, sewn thread, rivets, and/or the like). In various embodiments, the connecting portions may be secured to a top side of the backing layer. However, it should be understood that in various embodiments, the connecting portions may be secured to the bottom side of the backing layer, while enabling a detachable handle to be secured relative to a top side of the backing layer. For example, one or more magnets may be secured to a bottom side of the backing layer (e.g., below adhesive layer or between adhesive layer and backing layer) to enable a detachable handle to be secured relative to a top side of the backing layer. In yet other embodiments, the backing layer may comprise a plurality of layers (e.g., two layers) secured together (e.g., with one or more fasteners) in such embodiments, the one or more connecting portions may be secured between two or more of the layers of the backing material.

Moreover, in embodiments in which the therapy tape comprises one or more stimulators, the stimulators may be secured relative to a bottom side of the therapy tape (e.g., below adhesive layer and/or between adhesive layer and backing layer). In yet other embodiments, the stimulators may be secured relative to a top side of the therapy tape. In yet other embodiments, one or more apertures may be formed within the therapy tape extending through the top side and bottom side of the therapy tape, and the one or more stimulators may be secured within the formed apertures. Moreover, a conduit (e.g., electrical conduit and/or pneumatic conduit, may be secured relative to each of the one or more stimulators. Moreover, a controller may be secured relative to the backing material (e.g., relative to a top side of the backing material, relative to a bottom side of the backing material, or relative to an end of the backing material), and the conduit may be secured relative to the controller. In various embodiments, the therapy tape comprising the one or more stimulators may be cut to a predefined length (e.g., 6 inches, 12 inches, 36 inches, and/or the like) and a controller may be secured relative to each length of therapy tape. In various embodiments, a protective sheet (e.g., a paper sheet, a wax paper sheet, a foil, and/or the like) may be secured relative to the adhesive layer to impede the adhesive layer from unintentionally becoming adhered to one or more surfaces.

### Method of Use

As discussed herein, various embodiments of the described therapy tape may be utilized to provide therapeutic treatment of one or more ailments of a patient. For example, the described therapy tape may be configured to increase blood and/or other fluid flow to various portions of a patient's body, to relieve muscle pressure in various portions of a patient's body, to discourage formation of scar tissue, and/or the like.

In use, therapy tape having one or more handles secured thereto may be adhered to a patient's skin. In various embodiments, a length of therapy tape may be removed from a roll of therapy tape (e.g., by cutting a selectable length of therapy tape from the roll of therapy tape) and may be adhered to a patient's skin. In embodiments in which the adhesive layer of the therapy tape is heat activated by a patient's body heat after application of the tape to the patient.

Once the therapy tape is securely adhered to the patient's skin, a tensile force may be applied to one or more of the handles (e.g., by pulling one or more of the handles) to lift a portion of the patient's skin adjacent to the one or more handles. In various embodiments, the tensile force may be applied at least substantially normal to the surface of the patient's skin (e.g., at least substantially perpendicular to the patient's skin), however the tensile force may be applied at an acute angle relative to the patient's skin (e.g., between 0-90 degrees relative to the patient's skin). As just one non-limiting example, the tensile force may be applied at an angle between about 45-90 degrees relative to the patient's skin. The tensile force may be applied as a part of a multi-way skin and/or tissue massage and/or manipulation treatment. For example, the handles may be pulled and/or twisted and the tape may be compressed during the treatment. For example, the handles may be pulled in any direction (e.g., in a direction aligned at least in part with the length of the tape, in a direction aligned at least in part with the width of the tape, in a direction normal to the tape, and/or any directions in between). The one or more handles may also be twisted (e.g., around an axis normal to the tape, around an axis parallel with the length of the tape, around an axis parallel with the width of the tape, and/or any axis in between).

In embodiments comprising detachable handles and a connecting portion, the therapy tape may be applied as discussed above. Once applied to a patient's skin, one or more of the detachable handles may be secured relative to one or more connecting portions. Once secured, a tensile force may be applied to one or more of the detachable handles (e.g., by pulling one or more of the detachable handles) to lift the therapy tape and an adjacent portion of the patient's skin.

Moreover, in embodiments comprising one or more stimulators, the therapy tape comprising the one or more stimulators may be applied to a patient's skin. Once applied, the one or more stimulators may be activated by receiving an activation signal from the controller. In various embodiments, the controller may comprise a user interface disposed thereon (e.g., power switch, intensity selector, and/or the like), and accordingly the one or more stimulators may be activated by the controller in response to receipt of user input by the controller. Moreover, in certain embodiments, the controller may be in electronic communication (e.g., wired and/or wireless) with one or more external computing entities (e.g. handheld computing entities, user computing entities, and/or the like). In various embodiments, the one or more electronic computing entities may be configured to generate and transmit one or more electronic signals to the controller to cause the controller to activate or otherwise control the one or more stimulators. For example, a user may provide user input to the external computing entity indicative of a desired power level (e.g., vibration intensity, TENS signal intensity, pressure application intensity, and/or the like). In response to receipt of the user input, the external computing entity may transmit a signal to the controller of the therapy tape, causing the controller to generate and transmit corresponding signals to the one or more stimulators causing the stimulators to activate.

Moreover, as discussed herein, the therapy tape comprising the one or more stimulators may additionally comprise one or more handles (previously secured thereto and/or detachable). Accordingly, a tensile force may be applied to the one or more handles before, during, and/or after activating one or more stimulators embodied within the therapy tape.

### Conclusion

Many modifications and other embodiments of the inventions set forth herein will come to mind to one skilled in the art to which these inventions pertain having the benefit of the teachings presented in the foregoing descriptions and the associated drawings. Therefore, it is to be understood that the inventions are not to be limited to the specific embodiments disclosed and that modifications and other embodiments are intended to be included within the scope of the appended claims. Although specific terms are employed herein, they are used in a generic and descriptive sense only and not for purposes of limitation.

As discussed herein, the therapy tape may be usable with human patients (e.g., children, adolescents, adults, elderly, and/or the like). Certain embodiments may be usable with animal patients (e.g., horses, cats, dogs, and/or the like). Moreover, as discussed herein, various embodiments may be applied directly to a patient's skin. However, in various embodiments, therapy tape may be embedded within and/or on clothing (e.g., compression fit clothing, loose-fit clothing, smart clothing (e.g., having one or more network connected devices embedded therein), support devices (e.g., support sleeves, and/or the like), and/or the like.

## Claims

1. A fastening tape system comprising:
one or more fastening tapes, each of the one or more fastening tapes comprising:
a backing layer configured to conform to a portion of a patient's body,
wherein the flexible backing layer defines a top side and a bottom side opposite the top side; and
an adhesive material secured relative to the bottom side of the flexible backing layer, wherein the adhesive material is configured to adhere the backing layer against the patient's skin;
wherein the adhesive material is configured to maintain adherence with the backing layer and the patient's skin;
at least one stimulator configured to apply a stimulating signal to the patient's skin, wherein the stimulating signal is configured to cause a mechanical movement of the patient's skin in at least one dimension; and
one or more fastening means at least partially secured relative to the one or more fastening tapes.

2. The fastening tape system of claim 1, further comprising one or more handles secured relative to the top side of the flexible backing layer, wherein the one or more handles are secured to the flexible backing layer via one or more fasteners.

3. The fastening tape system of claim 1, wherein the mechanical movement comprises at least one of
a back-and-forth movement in one of a horizontal direction and vertical direction along an axis in the one dimension,
a movement defined in two dimensions, and
a mechanical rotational movement.

4. The fastening tape system of any of claims 1 - 3, wherein the stimulating signal is configured to cause a vibration of the patient's skin.

5. The fastening tape system of any of claims 1 - 4 wherein the at least one stimulator comprises an electrical stimulator comprising a Transcutaneous Electrical Neuro Stimulator (TENS), and/or a pressure applicator configured to cause a vertical movement in a vertical direction of an axis in one dimension or to cause a continuous pressure downwards.

6. The fastening tape system of any of claims 1 - 5, wherein said one or more stimulator(s) is/are fastened to the one or more fastening tapes at one or more fastening points.

7. The fastening tape system of any of claims 1 -6, wherein one or both of the fastening tape and the at least one stimulator has a curved profile corresponding at least in part to a profile of at least a portion of a patient's body.

8. The fastening tape system of any of claims 1 - 7, wherein the one or more fastening means is secured relative to the one or more fastening tapes by an adhesive, which preferably is heat activated by the patient's body heat.

9. The fastening tape system of claim 1, wherein the backing layer is inelastic or elastic.

10. The fastening tape system of claim 1, wherein the one or more fastening means are configured for securing one or more of the at least one stimulators to the one or more fastening tapes.

11. The fastening tape system of claim 2, wherein the one or more fasteners comprise a second adhesive material different from said adhesive material, wherein the second adhesive material is configured to permanently secure the one or more handles relative to the top side of the flexible backing layer.

12. The fastening tape system of claim 2, wherein the one or more fasteners comprise thread sewn through at least a portion of each of the one or more handles and the backing layer.

13. The fastening tape system of claim 2, wherein at least one of the one or more handles comprises a single-ply flexible sheet secured relative to the backing layer.

14. The fastening tape system of claim 2, wherein the one or more handles comprise a base portion configured to be secured onto the top side of the backing layer and a grip portion extending away from the base portion, and wherein the base portion of the one or more handles are secured relative to the top side of the backing layer via the one or more fasteners.

15. The fastening tape system of claim 2, wherein the backing layer defines a length and a width measured perpendicular to the length, wherein the length is substantially longer than the width, and wherein:
at least one of the one or more handles extends across the backing layer in a direction parallel with the width of the backing layer or with the the length of the backing layer..

16. The fastening tape system of claim 2, wherein the one or more handles are detachably secured relative to the backing layer, and wherein the one or more fasteners are selected from: magnets, hook-and-loop fasteners, or snap-fasteners.

17. The fastening tape system of any of claims 1 - 16, further comprising a controller configured to generate one or more stimulator signals to selectively activate the at least one stimulator; wherein the controller comprises at least one communication interface configured to receive data transmitted from at least one external computing entity, wherein the communication interface preferably is a wireless communication interface.

18. The fastening tape system of claim 16 or 17, further comprising at least one sensor configured for measurement of at least one property of the skin, wherein at least one sensor is further configured to transmit measurements results to the controller or to a mobile device, and wherein the controller is configured to adjust the one or more stimulator signals based at least in part on the measurements results.

19. The fastening tape of any of claims 16 - 18, wherein at least a portion of the at least one stimulating signal can be programmed
to start and stop automatically in accordance with a measurement signal of one or more of an oedema sensor and a strain gauge, and/or
either automatically or adjusted as a response to a sensor measurement for a certain duration, to be maintained for a certain duration at given intervals, and/or or for a number of durations.

20. The fastening tape of any of claims 16 - 19, wherein the one or more sensors include one or more of a strain gauge, an oedema sensor for measuring one or more of liquid content and fat content, a temperature sensor, an ultraviolet (UV) sensor, and infrared (IR) sensor, an audio sensor, a force sensor, a radio frequency (RF) sensor, an electrical sensor for measurement of one or more electrical properties, a mechanical sensor for measurement of one or more mechanical properties, a lymph flow sensor for measurement of lymph flow, a blood circulation sensor for measurement of blood circulation, an evaporation sensor for measurement of evaporation ability, a pH sensor, a position sensor for the rise of the skin, and an input sensor configured for registering an input signal given by the patient in order to increase or decrease the efficiency of a treatment for an adjustment of the treatment.

## Patentansprüche

1. Befestigungsbandsystem umfassend:
ein oder mehrere Befestigungsbänder, wobei das eine oder die mehreren Befestigungsbänder jeweils Folgendes umfassen:
eine Trägerschicht, die dazu ausgelegt ist, sich an die Form eines Körperbereichs eines Patienten anzupassen, wobei die flexible Trägerschicht eine Oberseite und eine der Oberseite entgegengesetzte Unterseite definiert; und
einen Klebstoff, der in Bezug auf die Unterseite der flexiblen Trägerschicht angebracht ist, wobei der Klebstoff dazu ausgelegt ist, die Trägerschicht an der Haut des Patienten anzukleben;
wobei der Klebstoff dazu ausgelegt ist, die Haftung mit der Trägerschicht und der Haut des Patienten aufrechtzuerhalten;
mindestens einen Stimulator, der dazu ausgelegt ist, ein Stimulationssignal an der Haut des Patienten anzulegen, wobei das Stimulationssignal dazu ausgelegt ist, eine mechanische Bewegung der Haut des Patienten in mindestens einer Dimension zu bewirken; und
ein oder mehrere Befestigungsmittel, die mindestens teilweise in Bezug auf das eine oder die mehreren Befestigungsbänder angebracht sind.

2. Befestigungsbandsystem nach Anspruch 1, das ferner einen oder mehrere Griffe umfasst, die in Bezug auf die Oberseite der flexiblen Trägerschicht angebracht sind, wobei der eine oder die mehreren Griffe über ein oder mehrere Befestigungselemente an der flexiblen Trägerschicht angebracht sind.

3. Befestigungsbandsystem nach Anspruch 1, wobei die mechanische Bewegung eines von Folgendem umfasst:
eine Hin- und Herbewegung in einer horizontalen Richtung oder in einer vertikalen Richtung entlang einer Achse in der einen Dimension,
eine Bewegung, die in zwei Dimensionen definiert ist, und
eine mechanische Drehbewegung.

4. Befestigungsbandsystem nach einem der Ansprüche 1 bis 3, wobei das Stimulationssignal dazu ausgelegt ist, eine Vibration der Haut des Patienten zu bewirken.

5. Befestigungsbandsystem nach einem der Ansprüche 1 bis 4, wobei der mindestens eine Simulator Folgendes umfasst: einen elektrischen Stimulator, der einen transkutanen elektrischen Neurostimulator (TENS) umfasst, und/oder einen Druckapplikator, der dazu ausgelegt ist, eine vertikale Bewegung in einer vertikalen Richtung einer Achse in einer Dimension zu bewirken oder einen kontinuierlichen Druck nach unten zu bewirken.

6. Befestigungsbandsystem nach einem der Ansprüche 1 bis 5, wobei der eine oder die mehreren Stimulator(en) an einem oder mehreren Befestigungspunkten an dem einen oder den mehreren Befestigungsbändern befestigt ist/sind.

7. Befestigungsbandsystem nach einem der Ansprüche 1 bis 6, wobei das Befestigungsband und/oder der mindestens eine Stimulator ein gekrümmtes Profil aufweist, das mindestens teilweise einem Profil mindestens eines Körperbereichs eines Patienten entspricht.

8. Befestigungsbandsystem nach einem der Ansprüche 1 bis 7, wobei das eine oder die mehreren Befestigungsmittel in Bezug auf das eine oder die mehreren Befestigungsbänder über einen Kleber angebracht sind, der vorzugsweise durch die Körperwärme des Patienten wärmeaktiviert wird.

9. Befestigungsbandsystem nach Anspruch 1, wobei die Trägerschicht unelastisch oder elastisch ist.

10. Befestigungsbandsystem nach Anspruch 1, wobei das eine oder die mehreren Befestigungsmittel dazu ausgelegt sind, einen oder mehrere der mindestens einen Stimulatoren an dem einen oder den mehreren Befestigungsbändern anzubringen.

11. Befestigungsbandsystem nach Anspruch 2, wobei das eine oder die mehreren Befestigungselemente einen zweiten Klebstoff umfassen, der sich vom vorgenannten Klebstoff unterscheidet, wobei der zweite Klebstoff dazu ausgelegt ist, den einen oder die mehreren Griffe in Bezug auf die Oberseite der flexiblen Trägerschicht dauerhaft anzubringen.

12. Befestigungsbandsystem nach Anspruch 2, wobei das eine oder die mehreren Befestigungselemente einen Faden umfassen, der durch mindestens einen Abschnitt eines jeden der einen oder mehreren Griffe und die Trägerschicht hindurchgenäht ist.

13. Befestigungsbandsystem nach Anspruch 2, wobei mindestens einer der einen oder mehreren Griffe ein in Bezug auf die Trägerschicht angebrachtes einlagiges flexibles Flächenelement umfasst.

14. Befestigungsbandsystem nach Anspruch 2, wobei der eine oder die mehreren Griffe einen Basisabschnitt, der dazu ausgelegt ist, auf der Oberseite der Trägerschicht angebracht zu werden, und einen Griffabschnitt, der sich vom Basisabschnitt weg erstreckt, umfassen, und wobei der Basisabschnitt des einen oder der mehreren Griffe über das eine oder die mehreren Befestigungselemente in Bezug auf die Oberseite der Trägerschicht angebracht ist.

15. Befestigungsbandsystem nach Anspruch 2, wobei die Trägerschicht eine Länge und eine rechtwinklig zur Länge gemessene Breite definiert, wobei die Länge im Wesentlichen länger als die Breite ist, und wobei:
mindestens einer der einen oder mehreren Griffe sich quer über die Trägerschicht in einer parallel zur Breite der Trägerschicht oder zur Länge der Trägerschicht verlaufenden Richtung erstreckt.

16. Befestigungsbandsystem nach Anspruch 2, wobei der eine oder die mehreren Griffe in Bezug auf die Trägerschicht lösbar angebracht sind, und wobei das eine oder die mehreren Befestigungselemente ausgewählt sind aus: Magneten, Klettverschlüssen oder Druckknöpfen.

17. Befestigungsbandsystem nach einem der Ansprüche 1 bis 16, ferner umfassend: ein Steuergerät, das dazu ausgelegt ist, ein oder mehrere Stimulatorsignale zu erzeugen, um den mindestens einen Stimulator selektiv zu aktivieren; wobei das Steuergerät mindestens eine Kommunikationsschnittstelle umfasst, die dazu ausgelegt ist, Daten zu empfangen, die von mindestens einer externen Recheneinheit gesendet werden, wobei die Kommunikationsschnittstelle vorzugsweise eine drahtlose Kommunikationsschnittstelle ist.

18. Befestigungsbandsystem nach Anspruch 16 oder 17, das ferner mindestens einen Sensor umfasst, der dazu ausgelegt ist, mindestens eine Eigenschaft der Haut zu messen, wobei mindestens ein Sensor ferner dazu ausgelegt ist, Messergebnisse an das Steuergerät oder an eine mobile Vorrichtung zu senden, und wobei das Steuergerät dazu ausgelegt ist, das eine oder die mehreren Stimulatorsignale mindestens teilweise auf Grundlage der Messergebnisse anzupassen.

19. Befestigungsband nach einem der Ansprüche 16 bis 18, wobei mindestens ein Teil des mindestens einen Stimulationssignals so programmiert sein kann,
dass es sich in Abhängigkeit von einem Messsignal eines Ödemsensors und/oder Dehnungsmessers automatisch zuschaltet oder abschaltet, und/oder
dass es, entweder automatisch oder in angepasster Antwort auf eine Sensormessung über eine bestimmte Dauer, für einen bestimmten Zeitraum in gegebenen Intervallen und/oder für eine Anzahl von Zeiträumen aufrechterhalten wird.

20. Befestigungsband nach einem der Ansprüche 16 bis 19, wobei der eine oder die mehreren Sensoren eines oder mehreres von Folgendem umfassen: einen Dehnungsmesser, einen Ödemsensor zur Messung von Flüssigkeitsgehalt und/oder Fettgehalt, einen Temperatursensor, einen Ultraviolett-(UV)-Sensor und Infrarot-(IR)-Sensor, einen Schallsensor, einen Kraftsensor, einen Hochfrequenz-(RF)-Sensor, einen elektrischen Sensor zur Messung einer oder mehrerer elektrischer Eigenschaften, einen mechanischen Sensor zur Messung einer oder mehrerer mechanischer Eigenschaften, einen Lymphflusssensor zur Messung des Lymphflusses, einen Blutflusssensor zur Messung des Blutflusses, einen Verdunstungssensor zur Messung der Verdunstungsfähigkeit, einen pH-Sensor, einen Lagesensor für die Erhebung der Haut, und einen Eingangssensor, der dazu ausgelegt ist, ein vom Patienten gegebenes Eingangssignal aufzunehmen, um zur Anpassung der Behandlung die Effizienz einer Behandlung zu erhöhen oder zu verringern.

## Revendications

1. Système de ruban de fixation comprenant :
un ou plusieurs rubans de fixation, chacun desdits un ou plusieurs rubans de fixation comprenant :
une couche de support configurée pour se conformer à une partie du corps d'un patient, ladite couche de support flexible définissant une face supérieure et une face inférieure opposée à la face supérieure; et
un matériau adhésif attaché par rapport à la face inférieure de la couche de support flexible, ledit matériau adhésif étant configuré pour faire adhérer la couche de support à la peau du patient;
dans lequel le matériau adhésif est configuré pour maintenir l'adhérence avec la couche de support et la peau du patient;
au moins un stimulateur configuré pour appliquer un signal stimulant à la peau du patient, ledit signal stimulant étant configuré pour provoquer un mouvement mécanique de la peau du patient dans au moins une dimension; et
un ou plusieurs moyens de fixation au moins partiellement attachés par rapport auxdits un ou plusieurs rubans de fixation.

2. Système de ruban de fixation selon la revendication 1, comprenant également une ou plusieurs poignées attachées par rapport à la face supérieure de la couche de support flexible, lesdites une ou plusieurs poignées étant attachées à la couche de support flexible par le biais d'un ou plusieurs éléments de fixation.

3. Système de ruban de fixation selon la revendication 1, dans lequel le mouvement mécanique comprend au moins l'un parmi
un mouvement de va-et-vient dans une direction horizontale ou une direction verticale le long d'un axe dans ladite une dimension,
un mouvement défini en deux dimensions, et
un mouvement de rotation mécanique.

4. Système de ruban de fixation selon l'une des revendications 1 à 3, dans lequel le signal stimulant est configuré pour provoquer une vibration de la peau du patient.

5. Système de ruban de fixation selon l'une des revendications 1 à 4, dans lequel ledit au moins un stimulateur comprend un stimulateur électrique comprenant un neurostimulateur électrique transcutané (TENS) et/ou un applicateur de pression configuré pour provoquer un mouvement vertical dans une direction verticale d'un axe dans une dimension ou pour provoquer une pression continue vers le bas.

6. Système de ruban de fixation selon l'une des revendications 1 à 5, dans lequel lesdits un ou plusieurs stimulateur(s) est/sont fixé(s) auxdits un ou plusieurs rubans de fixation en un ou plusieurs points de fixation.

7. Système de ruban de fixation selon l'une des revendications 1 à 6, dans lequel le ruban de fixation et/ou ledit au moins un stimulateur a un profil incurvé correspondant au moins en partie au profil d'au moins une partie du corps d'un patient.

8. Système de ruban de fixation selon l'une des revendications 1 à 7, dans lequel lesdits un ou plusieurs moyens de fixation sont attachés par rapport auxdits un ou plusieurs rubans de fixation par un adhésif qui est préférablement thermoactivé par la chaleur corporelle du patient.

9. Système de ruban de fixation selon la revendication 1, dans lequel la couche de support est inélastique ou élastique.

10. Système de ruban de fixation selon la revendication 1, dans lequel lesdits un ou plusieurs moyens de fixation sont configurés pour fixer un ou plusieurs desdits au moins un stimulateurs auxdits un ou plusieurs rubans de fixation.

11. Système de ruban de fixation selon la revendication 2, dans lequel lesdits un ou plusieurs éléments de fixation comprennent un deuxième matériau adhésif différent dudit matériau adhésif, ledit deuxième matériau adhésif étant configuré pour fixer de manière permanente lesdites une ou plusieurs poignées par rapport à la face supérieure de la couche de support flexible.

12. Système de ruban de fixation selon la revendication 2, dans lequel lesdits un ou plusieurs éléments de fixation comprennent un fil cousu à travers au moins une partie de chacune desdites une ou plusieurs poignées et la couche de support.

13. Système de ruban de fixation selon la revendication 2, dans lequel au moins l'une desdites une ou plusieurs poignées comprend une feuille monocouche flexible attachée par rapport à la couche de support.

14. Système de ruban de fixation selon la revendication 2, dans lequel lesdites une ou plusieurs poignées comprennent une partie de base configurée pour être attachée à la face supérieure de la couche de support et une partie de préhension s'étendant de la partie de base, et dans lequel la partie de base desdites une ou plusieurs poignées est attachée par rapport à la face supérieure de la couche de support par le biais desdits un ou plusieurs éléments de fixation.

15. Système de ruban de fixation selon la revendication 2, dans lequel la couche de support définit une longueur et une largeur mesurée perpendiculairement à la longueur, la longueur étant essentiellement plus longue que la largeur, et dans lequel :
au moins l'une desdites une ou plusieurs poignées s'étend en travers de la couche de support dans une direction parallèle à la largeur de la couche de support ou à la longueur de la couche de support.

16. Système de ruban de fixation selon la revendication 2, dans lequel lesdites une ou plusieurs poignées sont attachées de manière amovible par rapport à la couche de support, et dans lequel lesdits un ou plusieurs éléments de fixation sont choisis parmi : les aimants, fermetures autoagrippantes ou boutons-pression.

17. Système de ruban de fixation selon l'une des revendications 1 à 16, comprenant également un contrôleur configuré pour générer un ou plusieurs signaux stimulateurs afin d'activer sélectivement ledit au moins un stimulateur; dans lequel le contrôleur comprend au moins une interface de communication configurée pour recevoir des données transmises par au moins une entité informatique externe, l'interface de communication étant préférablement une interface de communication sans fil.

18. Système de ruban de fixation selon la revendication 16 ou 17, comprenant également au moins un capteur configuré pour mesurer au moins une propriété de la peau, dans lequel au moins un capteur est également configuré pour transmettre des résultats de mesure au contrôleur ou à un dispositif mobile, et dans lequel le contrôleur est configuré pour ajuster lesdits un ou plusieurs signaux stimulateurs au moins en partie sur la base des résultats de mesure.

19. Ruban de fixation selon l'une des revendications 16 à 18, dans lequel au moins une partie dudit au moins un signal stimulant peut être programmée
pour démarrer et s'arrêter automatiquement en fonction d'un signal de mesure provenant d'un capteur d'œdème et/ou d'une jauge extensométrique, et/ou
pour être, soit automatiquement, soit ajusté en réponse à une mesure du capteur pendant une certaine durée, maintenu pendant une certaine durée à des intervalles donnés, et/ou pendant un nombre de durées.

20. Ruban de fixation selon l'une des revendications 16 à 19, dans lequel lesdits un ou plusieurs capteurs comprennent l'un ou plusieurs parmi une jauge extensométrique, un capteur d'œdème pour mesurer une teneur en liquide et/ou une teneur en graisse, un capteur de température, un capteur ultraviolet (UV) et un capteur infrarouge (IR), un capteur audio, un capteur de force, un capteur de radiofréquence (RF), un capteur électrique pour mesurer une ou plusieurs propriétés électriques, un capteur mécanique pour mesurer une ou plusieurs propriétés mécaniques, un capteur de flux lymphatique pour mesurer le flux lymphatique, un capteur de circulation sanguine pour mesurer la circulation sanguine, un capteur d'évaporation pour mesurer la capacité d'évaporation, un capteur de pH, un capteur de position pour la remontée de la peau, et un capteur d'entrée configuré pour enregistrer un signal d'entrée donné par le patient afin d'augmenter ou de diminuer l'efficacité d'un traitement pour ajuster le traitement.
